# EUROPÄISCHE PATENTSCHRIFT

(11) **EP 3 206 756 B1**
(45) Veröffentlichungstag und Bekanntmachung des Hinweises auf die Patenterteilung: **19.09.2018**
(21) Anmeldenummer: 15778692.2
(22) Anmeldetag: 13.10.2015
(51) Int. Cl.: A61Q 13/00, A61L 9/01, C11D 3/50, C11B 9/00, C07C 45/82

(54) **VERWENDUNG VON HEXADECA-8,15-DIENAL ALS AROMACHEMIKALIE**
USE OF HEXADECA-8,15-DIENAL AS AN AROMA CHEMICAL
UTILISATION DE HEXADÉCANE-8,15-DIÉNAL COMME PRODUIT CHIMIQUE AROMATIQUE

(30) Priorität: 14.10.2014 EP 14188771
(43) Veröffentlichungstag der Anmeldung: 23.08.2017
(73) Patentinhaber: BASF SE, 67056 Ludwigshafen (DE)
(72) Erfinder: PELZER, Ralf, 37699 Fürstenberg (DE); THRUN, Frauke, 68163 Mannheim (DE); TELES, Joaquim Henrique, 67165 Waldsee (DE); WERNER, Albert, 67227 Frankenthal (DE); MAURER, Stephan, 67435 Neustadt (DE)
(86) Internationale Anmeldenummer: PCT/EP2015/073662
(87) Internationale Veröffentlichungsnummer: WO 2016/059042

(56) Entgegenhaltungen:
- EP-A1- 0 376 888
- US-A1- 2010 191 018

## Beschreibung

Die vorliegende Erfindung betrifft die Verwendung eines Gemisches von (E/Z)-Hexadeca-8,15-dienal oder eines Stoffgemisches, welches diese Verbindung enthält, als Aromachemikalie, insbesondere als Riechstoff; sowie Verfahren zu dessen Herstellung, außerdem Aromastoffzusammensetzungen und Mittel, die Hexadeca-8,15-dienal enthalten.

### Hintergrund der Erfindung

Trotz einer Vielzahl bereits vorhandener Riechstoffe besteht in der Parfümindustrie ein ständiger Bedarf an neuen Riechstoffen, die über ihre primären, nämlich geruchlichen (olfaktorischen) Eigenschaften hinaus zusätzliche positive Sekundäreigenschaften besitzen, wie z.B. eine effiziente Herstellungsweise, eine höhere Stabilität unter bestimmten Anwendungsbedingungen, eine höhere Ausgiebigkeit oder ein besseres Haftungsvermögen, oder aber durch Synergieeffekte mit anderen Riechstoffen zu besseren sensorischen Profilen führen.

Es besteht in der Parfümindustrie auch grundsätzlich ein Bedarf an weiteren Riechstoffen, die sich zur Herstellung von Riechstoffkompositionen und/oder parfümierten Artikeln eignen. Insbesondere besteht ein Bedarf an Riechstoffen, die durch die oben erwähnten technischen Eigenschaften zu einem erhöhten Nutzen in Riechstoffkompositionen führen. So können z.B. durch den Einsatz von Riechstoffen mit einer effizienten Herstellungsweise, einer höheren Stabilität und einem besseren sensorischen Profil die Einsatzmengen und die Anzahl von Riechstoffen in entsprechenden Formulierungen optimiert und/oder minimiert werden, was zu einer nachhaltigen Ressourcenschonung beim Parfümieren von Konsum- und Verbrauchsgütern führt.

Hauptinhaltsstoff von natürlich vorkommenden Moschus ist Muscon. Synthetisch hergestelltes Muscon ist ein Racemat also ein 1:1-Gemisch der zyklischen Ketone (*R*)-(-)-3-Methylcyclopentadecanon und (*S*)-(+)-3-Methylcyclopentadecanon.

Im Bereich der Riech- und Duftstoffe gibt es bereits eine Vielzahl von moschusartigen Komponenten, z. B. Habanolid, Exaltolid®, Muscenon und Globanon®. Diese Verbindungen gehören zur Klasse der gesättigten/ungesättigten Carbonyl haltigen Makrozyklen. Neben den zyklischen moschusartigen Duftstoffen gibt es keine bzw. nur wenige langkettige (Helvetolid®, Cyclomusk®) gesättigte und/oder ungesättigte Carbonylverbindungen, die diese olfaktorischen Eigenschaft besitzen.

Insbesondere besteht ein Bedarf an weiteren Riechstoffen und Riechstoffkompositionen mit einer Moschus-Note.

Die US2010/0191018 beschreibt die Oxidation verschiedener mehrfach ungesättigter cyclischer Aliphaten, wie z.B. Cyclohexadeca-1,9-dien, mit Distickstoffmonoxid. Als Nebenprodukt beobachtet man dabei die Bildung von Hexadeca-8,15-dienal. Dessen Eignung als Riechstoff mit Moschus-Charakter ist jedoch noch nicht beschrieben worden.

### Zusammenfassung der Erfindung

Überraschenderweise wurde obige Aufgabe durch Bereitstellung von Hexadeca-8,15-dienal gelöst. Die Isolierung eines Gemisches von (E/Z)-Hexadeca-8,15-dienal gelang überraschenderweise aus dem Reaktionsgemisch der Oxidation von Cyclohexadecadien mit Distickstoffmonoxid. Das Gemisch zeichnet sich durch einen ausgeprägten Moschusduft aus.

### Detaillierte Beschreibung der Erfindung

### a) Allgemeine Definitionen:

"Hexadeca-8,15-dienal" im Sinne der Erfindung umfasst sowohl eine der beiden stereoisomeren Formen (d.h. die 8-(E)- als auch die 8-(Z)-Form) einzeln oder als Gemisch dieser beiden Stereoisomeren ((E/Z)-Hexadeca-8,15-dienal), aber auch Stoffgemischen, welche "im Wesentlichen", d.h. mit einem Gewichtsanteil von mehr als 90%, insbesondere mehr als 95% und vor allem mehr als 96, 97, 98 oder 99% "Hexadeca-8,15-dienal" in stereoisomerenreiner Form oder als Stereoisomerengemisch enthalten. Werden keine andere Angaben gemacht, so bedeutet "Hexadeca-8,15-dienal" im Kontext der Erfindung ein Stereoisomerengemisch der 8-(E)- als auch die 8-(Z)-Form, vorzugsweise in einem molaren Verhältnis von E- zu Z von etwa 1:5 bis 5:1, wie z.B. etwa 1:2 bis 2:1 oder insbesondere etwa 1:1.

Die 8-(E)-Form besitzt folgende Struktur:

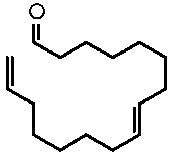

Eine "Aromachemikalie" ist ein Oberbegriff für Verbindungen, die als "Riechstoff" und/oder als "Geschmacksstoff" einsetzbar sind.

Als "Riechstoff" sind im Sinne der vorliegenden Erfindung natürliche oder synthetische Stoffe mit Eigengeruch zu verstehen.

Als "Geschmacksstoff" sind im Sinne der vorliegenden Erfindung natürliche oder synthetische Stoffe mit Eigengeschmack zu verstehen.

Der "Geruch" oder die "olfaktorische Wahrnehmung" ist im Sinne der vorliegenden Erfindung die Interpretation der Sinneserregungen, die von den Chemorezeptoren der Nase oder anderen Geruchsorganen an das Gehirn eines Lebewesens geliefert werden. Der Geruch kann folglich eine Sinneswahrnehmung der Nase von Riechstoffen sein, die beim Einatmen erfolgt. Die Luft dient hierbei als Geruchsträger.

Als "Duft" ist im Sinne der vorliegenden Erfindung ein wohlriechender Geruch zu verstehen. Entsprechendes gilt für einen erfindungsgemäßen "Duftstoff".

Ein "Parfüm" ist im Sinne der vorliegenden Erfindung ein Gemisch aus Riechstoffen und Träger, wie insbesondere einem Alkohol.

Eine "Parfümzusammensetzung" ist im Sinne der vorliegenden Erfindung ein Parfüm, welches unterschiedliche Mengen harmonisch aufeinander abgestimmte Einzelkomponenten enthält. Die Eigenschaften der Einzelbestandteile werden genutzt, um in der Kombination ein neues Gesamtbild zu schaffen, wobei die Charakteristika der Ingredienzen in den Hintergrund treten, ohne jedoch unterdrückt zu werden.

Ein "Parfümöl" ist im Sinne der vorliegenden Erfindung ein konzentriertes Gemisch aus mehreren Riechstoffen, die z.B. in alkoholischen Lösungen zur Parfümierung verschiedener Produkte gebraucht werden.

Ein "Duftthema" ist im Sinne der vorliegenden Erfindung die vorherrschende Duftnote in einer Riechstoffzusammensetzung.

Unter einer "Moschus-Note" oder "Moschus-Duft" ist im Rahmen der vorliegenden Erfindung ein Geruch zu verstehen, der dem Geruch des natürlichen Moschus bzw. wie dem seiner Bestandteile ähnlich ist.

Die "Kopfnote" ist im Sinne der vorliegenden Erfindung die erste Phase des Duftablaufs eines Parfüms. Sie spielt die ausschlaggebende Rolle beim ersten Eindruck, beim Öffnen des Flakons und beim Auftragen des Parfüms auf die Haut. Die Aufgabe der Kopfnote ist es, Interesse für das Parfüm allgemein zu wecken und für Aufmerksamkeit zu sorgen. Deshalb ist ein außergewöhnlicher Charakter häufig wichtiger als eine ausgefeilte Harmonie. Die Kopfnote wird naturgemäß von leichtflüchtigen Riechstoffen bestimmt.

"Modifizieren" bedeutet im Sinne der vorliegenden Erfindung, das Grundthema einer Riechstoffzusammensetzung mit zusätzlichen oder anderen Akkorden und Geruchsnuancen zu versehen. "Akkorde" entstehen im Sinne der vorliegenden Erfindung durch das Zusammenfügen verschiedener Riechstoffe, die sich somit zu neuen Geruchsbildern vereinigen. Die Anzahl der eingesetzten Riechstoffe kann von zwei bis zu hundert verschiedenen reichen.

Eine "organoleptisch/sensorisch wirksame Menge" im Sinne der vorliegenden Erfindung ist die Menge eines Riechstoffes, die ausreicht, um anregend auf ein Sinnesorgan beziehungsweise anregend auf einen sensorischen Rezeptor zu wirken.

### b) Spezielle Ausführungsformen der Erfindung

Vorliegende Erfindung betrifft insbesondere folgende Ausführungsformen:
1. Verwendung von Hexadeca-8,15-dienal der Formel **I** 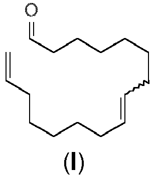 ((E/Z)-Hexadeca-8,15-dienal oder (E)-Hexadeca-8,15-dienal oder (Z)-Hexadeca-8,15-dienal) oder eines Stoffgemisches, welches diese Verbindung enthält, als Aromachemikalie, insbesondere als Riechstoff.
2. Verwendung nach Ausführungsform 1 in Mitteln, ausgewählt unter Parfüms, Wasch- und Reinigungsmitteln, kosmetischen Mitteln, Körperpflegemitteln, Hygieneartikeln, Lebensmitteln, Nahrungsergänzungsmitteln, Duftspendern, Duftstoffen, pharmazeutischen Mitteln und Pflanzenschutzmitteln.
3. Verwendung nach Ausführungsform 1 als Formulierungsmittel in Zubereitungen, die wenigstens einen Duft- und / oder wenigstens einen Aromastoff enthalten.
4. Verwendung von Verbindungen der Formel (**I**) zum Vermitteln, Modifizieren und/oder Verstärken einer Moschusduftnote in einer Riechstoffzusammensetzung durch Beimischen einer sensorisch wirksamen Menge wenigstens eines Stoffs oder eines Stoffgemisches gemäß der Definition in Ausführungsform 1.
5. Verwendung nach einer der vorhergehenden Ausführungsformen, wobei die Verbindung der Formel (**I**) in stereoisomerenreiner Form oder als E- und Z-Gemisch eingesetzt wird.
6. Verwendung nach einer der vorhergehenden Ausführungsformen, wobei ein Stereoisomerengemisch eingesetzt wird, worin der Gewichtsanteil von 8-(E)-Hexadeca-8,15-dienal, bezogen auf das Gesamtgewicht von (E)- und (Z)-Hexadeca-8,15-dienal, in einem Bereich von 1% bis weniger als 100%, wie z.B. in einem Bereich von 5% bis 95%, 15% bis 85%, 25% bis 75%, 40% bis 60%, 45% bis 55% oder bei etwa 50% liegt.
7. Verfahren zur Isolierung von Hexadeca-8,15-dienal wobei man aus einem Reaktionsgemisch, insbesondere aus einem Nebenproduktstrom der Oxidation von Cyclohexadeca-1,9-dien mit Lachgas (N₂O), der Hexadeca-8,15-dienal enthält, ein (E)- und (Z)-Gemisch von Hexadeca-8,15-dienal isoliert, wobei man Hexadeca-8,15-dienal als (E)- und (Z)-Gemisch mittels fraktionierter Destillation isoliert.
8. Verfahren nach Ausführungsform 7, wobei man Hexadeca-8,15-dienal als (E)- und (Z)-Gemisch aus einem Stoffgemisch isoliert, das eine Mischung aus (E/Z)-Hexadeca-8,15-dienal und Cyclohexadec-8-enon umfasst, worin der Gewichtsanteil von (E/Z)-Hexadeca-8,15-dienal, bezogen auf das Gesamtgewicht von (E/Z)-Hexadeca-8,15-dienal und Cyclo-hexadec-8-enon mindestens etwa 5%, wie z.B. etwa 10 bis 65% beträgt.
9. Verfahren nach Ausführungsform 7 oder 8, wobei man Hexadeca-8,15-dienal als (E)- und (Z)-Gemisch mittels einer chromatographischen Methode isoliert, wobei man Hexadeca-8,15-dienal als (E)- und (Z)-Gemisch aus einem Stoffgemisch isoliert, worin das Gewichtsanteil von (E/Z)-Hexadeca-8,15-dienal, bezogen auf das Gesamtgewicht des Stoffgemisches mindestens etwa 35%, wie z.B. etwa 40 bis 90% beträgt.
10. Mittel, enthaltend Hexadeca-8,15-dienal gemäß der Definition in einer der Ausführungsformen 1 bis 6, wobei das Mittel ausgewählt ist unter Parfüms, Wasch- und Reinigungsmitteln, kosmetischen Mitteln, Körperpflegemitteln, Hygieneartikeln, Lebensmitteln, Nahrungsergänzungsmitteln, Duftspendern, Duftstoffen, pharmazeutischen Mitteln und Pflanzenschutzmitteln.
11. Mittel nach Ausführungsform 10, enthaltend Hexadeca-8,15-dienal gemäß der Definition in einer der Ausführungsformen 1 bis 6 in einem Gewichtsanteil von 0,01 bis 99,9 Gew.-% wie z.B. etwa 10 bis 90, 15 bis 85, 25 bis 75, 40 bis 60, 45 bis 55 Gew.-%, bezogen auf das Gesamtgewicht der Zusammensetzung.
12. Verwendung von Hexadeca-8,15-dienal gemäß der Definition in einer der Ausführungsformen 1 bis 6 zum Vermitteln, Modifizieren und/oder Verstärken einer Moschusduftnote in einer Duft- oder Aromastoffzusammensetzung durch Beimischen einer sensorisch wirksamen Menge von Hexadeca-8,15-dienal.

### c) Weitere Ausgestaltungen der Erfindung

### c1) Riechstoffzusammensetzungen:

Einem weiteren Aspekt zufolge werden die erfindungsgemäß verwendeten Riechstoffe insbesondere zwecks effizienterer Handhabung und Dosierung, auch als Riechstoffmischungen mit Verdünnungs- oder Lösungsmitteln eingesetzt. Hierbei wird der Anteil der Riechstoffe, bezogen auf die Summe von Riechstoffen und Lösungsmittel, in Gew.-% angegeben.

### Lösungsmittel:

Ein "Lösungsmittel" dient im Sinne der vorliegenden Erfindung der Verdünnung der erfindungsgemäß zu verwendenden Riechstoffe oder der erfindungsgemäßen Riechstoffzusammensetzung, ohne eigene riechende Eigenschaften zu besitzen. Manche Lösungsmittel haben zugleich fixierende Eigenschaften.

Das Hexadeca-8,15-dienal kann zu 1 bis 99 Gew.-% einem Verdünnungs- oder Lösungsmittel beigemischt werden. Bevorzugt sind mindestens 40 Gew.-%ige Lösungen, weiter bevorzugt mindestens 50 Gew.-%ige Lösungen, weiterhin bevorzugt mindestens 60 Gew.-%ige Lösungen, weiter bevorzugt mindestens 70 Gew.-%ige Lösungen, insbesondere bevorzugt mindestens 80 Gew.-%ige Lösungen, weiterhin insbesondere bevorzugt mindestens 90 Gew.-%ige Lösungen, vorzugsweise in olfaktorisch akzeptablen Lösungsmitteln.

Bevorzugte olfaktorische akzeptable Lösungsmittel sind Ethanol, Dipropylenglycol (DPG), Propylenglycol, 1,2-Butylenglycol, Glycerin, Diethylenglycolmonoethylether, Diethylphthalat (DEP), Isopropylmyristat (IPM), Triethylcitrat (TEC), Benzylbenzoat (BB) und Benzylacetat. Hierbei wiederum bevorzugt sind Ethanol, Diethylphthalat, Propylenglycol, Dipropylenglycol, Triethylcitrat, Benzylbenzoat und Isopropylmyristat.

Eine "Riechstoffzusammensetzung" ist im Sinne der vorliegenden Erfindung eine Mischung, die neben Hexadeca-8,15-dienal mindestens einen weiteren Riechstoff umfasst. Insbesondere kann es sich bei einer solchen Riechstoffkomposition um eine Parfümkomposition (ein Parfümöl) handeln.

Erfindungsgemäße Riechstoffkompositionen enthalten, bezogen auf die Gesamtmenge der Riechstoffkomposition, z.B. eine Menge von Hexadeca-8,15-dienal von 0,01 bis 65 Gew.-%, vorzugsweise von etwa 0,1 bis etwa 50 Gew.-%, vorzugsweise von etwa 0,5 bis etwa 30 Gew.-% und besonders bevorzugt von etwa 0,5 bis etwa 25 Gew.-%. Das Gewichtsverhältnis von Hexadeca-8,15-dienal zu der Gesamtmenge an weiteren Riechstoffen liegt z.B. im Bereich von 1:1000 bis 1:0,5, vorzugsweise im Bereich von 1:700 bis 1:1, besonders bevorzugt im Bereich von 1:500 bis 1:10.

Erfindungsgemäße Riechstoffkompositionen enthalten, bezogen auf die Gesamtmenge der Riechstoffkomposition, z.B. eine Menge von Hexadeca-8,15-dienal von 0,01 bis 65 Gew.-%, vorzugsweise von etwa 0,1 bis etwa 50 Gew.-%, vorzugsweise von etwa 0,5 bis etwa 30 Gew.-% und besonders bevorzugt von etwa 0,5 bis etwa 25 Gew.-%. Das Gewichtsverhältnis von Hexadeca-8,15-dienal zu der Gesamtmenge an weiteren (von Hexadeca-8,15-dienal verschiedenen) Riechstoffen liegt z.B. im Bereich von 1:1000 bis 1:0,5, vorzugsweise im Bereich von 1:700 bis 1:1, besonders bevorzugt im Bereich von 1:500 bis 1:10.

### Weitere Riechstoffe:

Erfindungsgemäße Riechstoffkompositionen enthalten neben Hexadeca-8,15-dienal zumindest einen weiteren Riechstoff, vorzugsweise 2, 3, 4, 5, 6, 7, 8 oder weitere Riechstoffe, wobei weitere Riechstoffe z.B. ausgewählt sind unter:
Alpha-Hexylzimtaldehyd, 2-Phenoxyethylisobutyrat (Phenirat¹), Dihydromyrcenol (2,6-Dimethyl-7-octen-2-ol), Methyldihydrojasmonat (vorzugsweise mit einem Gehalt an cis-Isomerem von mehr als 60 Gew.-%) (Hedione⁹, Hedione HC⁹), 4,6,6,7,8,8-Hexamethyl-1,3,4,6,7,8-hexahydrocyclopenta[g]benzopyran (Galaxolid³), Tetrahydrolinalool (3,7-Dimethyloctan-3-ol), Ethyllinalool, Benzylsalicylat, 2-Methyl-3-(4-*tert*-butylphenyl)propanal (Lilial²), Zimtalkohol, 4,7-Methano-3a,4,5,6,7,7a-hexahydro-5-indenylacetat und/oder 4,7-Methano-3a,4,5,6,7,7a-hexahydro-6-indenylacetat (Herbaflorat¹), Citronellol, Citronellylacetat, Tetrahydrogeraniol, Vanillin, Linalylacetat, Styrolylacetat (1-Phenylethylacetat), Octahydro-2,3,8,8-tetramethyl-2-acetonaphthon und/oder 2-Acetyl-1,2,3,4,6,7,8-octahydro-2,3,8,8-tetramethyl-naphtalin (Iso E Super³), Hexylsalicylat, 4-*tert*-Butylcyclohexylacetat (Oryclone¹), 2-*tert*-Butylcyclohexylacetat (Agrumex HC¹), alpha-Ionon (4-(2,2,6-Trimethyl-2-cyclohexen-1-yl)-3-buten-2-on), n-alpha-Methylionon, alpha-Isomethylionon, Coumarin, Terpinylacetat, 2-Phenylethylalkohol, 4-(4-Hydroxy-4-methylpentyl)-3-cyclohexencarboxaldehyd (Lyral³), alpha-Amylzimtaldehyd, Ethylenbrassylat, (E)- und/oder (Z)-3-Methylcyclopentadec-5-enon (Muscenon⁹), 15-Pentadec-11-enolid und/oder 15-Pentadec-12-enolid (Globalide¹), 15-Cyclopentadecanolid (Macrolide¹), 1-(5,6,7,8-Tetrahydro-3,5,5,6,8,8-hexamethyl-2-naphthalenyl)-ethanon (Tonalid¹⁰), 2-Isobutyl-4-methyltetrahydro-2H-pyran-4-ol (Florol⁹), 2-Ethyl-4-(2,2,3-trimethyl-3-cyclopenten-1-yl)-2-buten-1-ol (Sandolen¹), *cis*-3-Hexenylacetat, *trans*-3-Hexenylacetat, *trans*-2/*cis*-6-Nonadienol, 2,4-Dimethyl-3-cyclohexencarboxaldehyd (Vertocitral¹), 2,4,4,7-Tetramethyl-oct-6-en-3-on (Claritone¹), 2,6-Dimethyl-5-hepten-1-al (Melonal²), Borneol, 3-(3-Isopropylphenyl)-butanal (Florhydral²), 2-Methyl-3-(3,4-methylendioxyphenyl)-propanal (Helional³), 3-(4-Ethylphenyl)-2,2-dimethylpropanal (Florazon¹), 7-Methyl-2H-1,5-benzodioxepin-3(4H)-on (Calone), 3,3,5-Trimethylcyclohexylacetat (vorzugsweise mit einem Gehalt an cis-Isomeren von 70 Gew.-%) oder mehr und 2,5,5-Trimethyl-1,2,3,4,4a,5,6,7-octahydronaphthalin-2-ol (Ambrinol S¹). Die vorangehend genannten Riechstoffe werden im Rahmen der vorliegenden Erfindung demnach bevorzugt mit erfindungsgemäßen Mischungen kombiniert.

Sofern vorstehend Handelsnamen angegeben sind, beziehen sich diese auf folgende Quellen:
¹ Handelsname Symrise GmbH, Deutschland;
² Handelsname Givaudan AG, Schweiz;
³ Handelsname International Flavors & Fragrances Inc., USA;
⁵ Handelsname Danisco Seillans S.A., Frankreich;
⁹ Handelsname Firmenich S.A., Schweiz;
¹⁰ Handelsname PFW Aroma Chemicals B.V., Niederlande.

Weitere Riechstoffe, mit denen Hexadeca-8,15-dienal z.B. zu einer Riechstoffkomposition kombiniert werden können, finden sich z.B. in S. Arctander, Perfume and Flavor Chemicals, Vol. I und II, Montclair, N. J., 1969, Selbstverlag oder K. Bauer, D. Garbe und H. Surburg, Common Fragrance and Flavor Materials, 4rd. Ed., Wiley- VCH, Weinheim 2001. Im Einzelnen seien genannt:
Extrakte aus natürlichen Rohstoffen wie etherischen Ölen, Concretes, Absolues, Resine, Resinoide, Balsame, Tinkturen wie z.B.
Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-Absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptuscitriodoraöl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen ;
Einzelriechstoffe aus der Gruppe der Kohlenwasserstoffe, wie z.B. 3-Caren; alpha-Pinen; beta-Pinen; alpha-Terpinen; gamma-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methylen-heptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; (E/Z)-1-(1-Methoxy-propoxy)-hex-3-en; der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadiensäurenitril; 3,7-Dimethyl-6-octensäurenitril;
der Ester von aliphatischen Carbonsäuren wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenylsobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
der acyclischen Terpenalkohole wie z.B. Geraniol; Nerol; Linalool; Lavandulol; Nerolidol; Farnesol; Tetrahydrolinalool; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z.B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal; der cyclischen Terpenalkohole wie z.B. Menthol; Isopulegol; alpha-Terpineol; Terpine-4-ol; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z.B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; 2-Methyl-4-(2,6,6-trimethyl-1-cyclohexen-1-yl)-2-butenal; Nootkaton; Dihydronootkaton; 4,6,8-Megastigmatrien-3-on; alpha-Sinensal ; beta-Sinensal ; acetyliertes Cedernholzöl (Methylcedrylketon);
der cyclischen Alkohole wie z.B. 4-*tert*-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyl-tetrahydro-2H-pyran-4-ol;
der cycloaliphatischen Alkohole wie z.B. alpha-3,3-Trimethylcyclohexylmethanol; 1-(4-Isopropylcyclohexyl)-ethanol; 2-Methyl-4-(2,2,3-trimethyl-3-cyclopent-1-yl)-butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6Trimethylcyclohexyl)hexan-3-ol;
der cyclischen und cycloaliphatischen Ether wie z.B. Cineol; Cedrylmethylether; Cyclododecylmethylether; 1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydro-naphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-*tert*-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-(cis-2-penten-1-yl)-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetra-methylcyclohexanon; 4-*tert*-Pentylcyclohexanon; 5-Cyclohexadecen-1-on; 6,7-Dihydro-1,1,2,3,3-pentamethyl-4(5H)-indanon; 8-Cyclohexadecen-1-on; 7-Cyclohexa-decen-1-on; (7/8)-Cyclohexadecen-1-on; 9-Cycloheptadecen-1-on; Cyclopentadecanon; Cyclohexadecanon;
der cycloaliphatischen Aldehyde wie z.B. 2,4-Dimethyl-3-cyclohexencarbaldehyd; 2-Methyl-4-(2,2,6-trimethylcyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z.B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; *tert*-Butyl-(2,4-dimethyl-3-cyclohexen-1-yl)-keton;
der Ester cyclischer Alkohole wie z.B. 2-*tert*-Butylcyclohexylacetat; 4-*tert*-Butylcyclohexylacetat; 2-*tert*-Pentylcyclohexylacetat; 4-*tert*-Pentylcyclohexylacetat; 3,3,5-Trimethylcyclohexylacetat; Decahydro-2-naphthylacetat; 2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5-, bzw. -6-indenylacetat; 4,7-Methano-3a,4,5,6,7,7a-hexahydro-5-, bzw. -6-indenylpropionat; 4,7-Methano-3a,4,5,6,7,7a-hexahydro-5-, bzw. -6-indenylisobutyrat; 4,7-Methanooctahydro-5-, bzw. -6-indenylacetat;
der Ester cycloaliphatischer Alkohole wie z.B. 1-Cyclohexylethylcrotonat;
der Ester cycloaliphatischer Carbonsäuren wie z.B. Allyl-3-cyclohexylpropionat; Allylcyclohexyloxyacetat; *cis-* und *trans*-Methyldihydrojasmonat; *cis-* und *trans*-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z.B. Benzylalkohol; 1-Phenylethylalkohol, 2-Phenylethylalkohol, 3-Phenylpropanol; 2-Phenylpropanol; 2-Phenoxyethanol; 2,2-Dimethyl-3-phenylpropanol; 2,2-Dimethyl-3-(3-methylphenyl)-propanol; 1,1-Dimethyl-2-phenylethylalkohol; 1,1-Dimethyl-3-phenylpropanol; 1-Ethyl-1-methyl-3-phenyl-propanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z.B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)-propanal; 2-Methyl-3-(4-*tert-*butylphenyl)-propanal; 2-Methyl-3-(4-isobutylphenyl)-propanal; 3-(4-*tert*-Butylphenyl)-propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)-propanal; 2-Methyl-3-(4-methylendioxyphenyl)-propanal;
der aromatischen und araliphatischen Ketone wie z.B. Acetophenon; 4 Methylacetophenon; 4-Methoxyacetophenon; 4-*tert*-Butyl-2,6-dimethylacetophenon; 4-Phenyl-2-butanon; 4-(4-Hydroxyphenyl)-2-butanon; 1-(2-Naphthalenyl)-ethanon; 2-Benzofuranylethanon; (3-Methyl-2-benzofuranyl)-ethanon; Benzophenon; 1,1,2,3,3,6-Hexamethyl-5-indanylmethylketon; 6*-tert-*Butyl-1,1-dimethyl-4-indanylmethylketon; 1-[2,3-Dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnamat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; *Cis*-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-*tert-*butylbenzol; 3,5-Dinitro-2,6-dimethyl-4-*tert*-butylacetophenon; Zimtsäurenitril; 3-Methyl-5-phenyl-2-pentensäurenitril; 3-Methyl-5-phenylpentansäurenitril; Methylanthranilat; Methyl-N-methylanthranilat; Schiff''sche Basen von Methylanthranilat mit 7-Hydroxy-3,7-dimethyloctanal, 2-Methyl-3-(4-*tert*-butylphenyl)-propanal oder 2,4-Dimethyl-3-cyclohexencarbaldehyd; 6-Isopropylchinolin; 6-Isobutylchinolin; 6-*sec*-Butylchinolin; 2-(3-Phenylpropyl)-pyridin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)-phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 4-Methyl-1,4-decanolid; 1,15-Pentadecanolid; *cis-* und *trans*-11-Pentadecen-1,15-olid; *cis-* und *trans-12-*Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

### c2) Riechstoffhaltige Artikel

Erfindungsgemäßes Hexadeca-8,15-dienal oder erfindungsgemäße Riechstoffkompositionen können in eine Reihe von Produkten eingearbeitet bzw. auf solche Produkte appliziert werden.

Erfindungsgemäße Riechstoffe können bei der Herstellung parfümierter Artikel eingesetzt. Die olfaktorischen Eigenschaften ebenso wie die stofflichen Eigenschaften (wie Löslichkeit in gängigen Lösungsmitteln und Kompatibilität mit gängigen weiteren Bestandteilen derartiger Produkte) sowie die toxikologische Unbedenklichkeit der erfindungsgemäßen Riechstoffe unterstreichen ihre besondere Eignung für die genannten Einsatzzwecke. Die positiven Eigenschaften tragen dazu bei, dass die erfindungsgemäß verwendeten Riechstoffe und die erfindungsgemäßen Riechstoffkompositionen besonders bevorzugt in Parfümerzeugnissen, Körperpflegeprodukten, Hygieneartikeln, Textilwaschmittel sowie in Reinigungsmitteln für feste Oberflächen eingesetzt werden.

Der parfümierte Artikel ist z.B. ausgewählt unter Parfümerzeugnissen, Körperpflegeprodukten, Hygieneartikeln, Textilwaschmitteln und Reinigungsmitteln für feste Oberflächen. Bevorzugte erfindungsgemäße parfümierte Artikel sind weiterhin ausgewählt unter:
Parfümerzeugnissen, ausgewählt unter Parfüm-Extrakten, Eau de Parfums, Eau de Toilettes, Eau de Colognes, Eau de Solide, Extrait Parfum, Luftverbesserern in flüssiger, gelartiger oder auf einem festen Träger aufgebrachter Form, Aerosolsprays, Duftreinigern und -ölen;
Körperpflegeprodukten, ausgewählt unter Rasierwässern, Pre-shave-Produkten, Splash-Colognes, festen und flüssigen Seifen, Duschgelen, Shampoos, Rasierseifen, Rasierschäumen, Badeölen, kosmetischen Emulsionen vom Öl-in-Wasser-, vom Wasser-in-ÖI- und vom Wasser-in-ÖI-in-Wasser-Typ wie z.B. Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigende Haarlotionen, Haarspülungen, Haarshampoo, permanenten und semipermanenten Haarfärbemitteln, Haarverformungsmitteln wie Kaltwellen und Haarglättungsmitteln, Haarwässern, Haarcremes und -lotionen, Deodorants und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara, Zahnpasta, Zahnseide;
Hygieneartikeln, ausgewählt unter Kerzen, Lampenölen, Räucherstäbchen, Insektiziden, Repellentien, Treibstoffen, Rostentfernern, parfümierten Erfrischungstüchern, Achselpads, Babywindeln, Damenbinden, Toilettenpapier, Kosmetiktüchern, Taschentüchern, Spülmaschinendeo;
Reinigungsmitteln für feste Oberflächen, ausgewählt unter parfümierten sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, Wachsen und Polituren wie Möbelpolituren, Fußbodenwachsen, Schuhcremes, Desinfektionsmitteln, Oberflächendesinfektionsmitteln und Sanitärreinigern, Bremsenreinigern, Rohrreinigern, Entkalkern, Grill- und Backofenreinigern, Algen- und Moosentfernern, Schimmelentfernern, Fassadenreinigungsmitteln;
Textilwaschmitteln, ausgewählt unter flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten.

Einem weiteren Aspekt zufolge sind die erfindungsgemäß verwendeten Riechstoffe und die erfindungsgemäßen Riechstoffkompositionen für den Einsatz in tensidhaltigen parfümierten Artikeln geeignet. Gesucht werden nämlich - insbesondere für die Parfümierung von tensidhaltigen Formulierungen wie zum Beispiel Reinigungsmitteln (insbesondere Geschirrspülmittel und Allzweckreiniger) - häufig Riechstoffe und/oder Riechstoffkompositionen mit einer Moschusnote und ausgeprägter Natürlichkeit.

Einem weiteren Aspekt zufolge können erfindungsgemäß verwendete Riechstoffe und erfindungsgemäße Riechstoffkompositionen als Mittel zum Versehen von (a) Haaren oder (b) textilen Fasern mit der Geruchsnote rosig verwendet werden.

Die erfindungsgemäß zu verwendenden Riechstoffe und erfindungsgemäße Riechstoffkompositionen eignen sich daher besonders gut für den Einsatz in tensidhaltigen parfümierten Artikeln.

Bevorzugt ist es, wenn der parfümierte Artikel einer der folgenden ist:
- ein saures, alkalisches oder neutrales Reinigungsmittel, das insbesondere aus der Gruppe ausgewählt ist bestehend aus Allzweckreinigern, Fußbodenreinigern, Fensterglasreinigern, Geschirrspülmitteln, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln,
- ein Luftverbesserer in flüssiger, gelartiger oder auf einem festen Träger aufgebrachter Form oder als Aerosolspray,
- ein Wachs oder eine Politur, die insbesondere aus der Gruppe ausgewählt ist bestehend aus Möbelpolituren, Fußbodenwachsen und Schuhcremes, oder
- ein Körperpflegemittel, das insbesondere aus der Gruppe ausgewählt ist bestehend aus Duschgelen und Shampoos, Rasierseifen, Rasierschäumen, Badeölen, kosmetischen Emulsionen vom Öl-in-Wasser-, vom Wasser-in-ÖI- und vom Wasser-in-ÖI-in-Wasser-Typ wie z.B. Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigen Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorants und Antiperspirantien wie z.B. Achselsprays, Roll-ons, Deosticks, Deocremes, Produkten der dekorativen Kosmetik.

Inhaltstoffe, mit denen erfindungsgemäß verwendete Riechstoffe oder erfindungsgemäße Riechstoffkompositionen vorzugsweise kombiniert werden können, sind beispielsweise: Konservierungsmittel, Abrasiva, Antiaknemittel, Mittel gegen Hautalterung, antibakterielle Mittel, Anticellulitismittel, Antischuppenmittel, entzündungshemmende Mittel, irritationsverhindernde Mittel, irritationshemmende Mittel, antimikrobielle Mittel, Antioxidantien, Adstringentien, schweisshemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Silicone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, alpha-Hydroxycarbonsäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate.

Einem weiteren Aspekt zufolge verwendet man die Riechstoffe bei der Herstellung der parfümierten Artikel in flüssiger Form, unverdünnt oder mit einem Lösungsmittel verdünnt oder in Form einer Riechstoffkomposition. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw. Für die genannten Lösungsmittel gilt, dass diese, im Falle des Vorhandenseins eigener olfaktorischer Eigenschaften, ausschließlich dem Bestandteil "Lösungsmittel" und nicht den "Riechstoffen" zuzuordnen sind.

Die in den erfindungsgemäßen parfümierten Artikeln enthaltenen Riechstoffe und/oder Riechstoffkompositionen können dabei in einer Ausführungsform an einem Trägerstoff absorbiert sein, der sowohl für eine feine Verteilung des Riechstoffs oder der Riechstoffkomposition im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose basierende Stoffe sein.

Die erfindungsgemäß verwendeten Riechstoffe und die erfindungsgemäßen Riechstoffkompositionen können auch mikroverkapselt, sprühgetrocknet, als Einschlusskomplexe oder als Extrusionsprodukte vorliegen und in dieser Form dem zu parfümierenden Produkt, bzw. Artikel, hinzugefügt werden. Die Eigenschaften können durch sogenanntes "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung kann beispielsweise durch das sogenannte Koazervationsverfahren mit Hilfe von Kapselmaterialien, z.B. aus polyurethanartigen Stoffen oder Weichgelatine, erfolgen. Die sprühgetrockneten Parfümöle können beispielsweise durch Sprühtrocknung einer das Parfümöl enthaltenden Emulsion bzw. Dispersion hergestellt werden, wobei als Trägerstoffe modifizierte Stärken, Proteine, Dextrin und pflanzliche Gummen verwendet werden können. Einschlusskomplexe können z.B. durch Eintragen von Dispersionen von Riechstoffkompositionen und Cyclodextrinen oder Harnstoffderivaten in ein geeignetes Lösungsmittel, z.B. Wasser, hergestellt werden. Extrusionsprodukte können durch Verschmelzen erfindungsgemäß verwendeter Riechstoffe und erfindungsgemäßer Riechstoffkompositionen mit einem geeigneten wachsartigen Stoff und durch Extrusion mit nachfolgender Erstarrung, ggf. in einem geeigneten Lösungsmittel, z.B. Isopropanol, hergestellt werden.

### c3) Isolierung erfindungsgemäßer Riechstoffe

### (E/Z)-Hexadeca-8,15-dienal ist z.B. aus der US20100191018 bekannt

Geeignete Quellen für die Isolierung von (E/Z)-Hexadeca-8,15-dienal ist der Nebenproduktstrom der N₂O-Oxidation von Cyclohexadeca-1,9-dien, wie z.B. beschrieben in der US20100191018.

Derartige Reaktionsprodukte sind dann in an sich bekannter Weise aufzuarbeiten, um das gewünschte Produkt in angereicherter Form oder in Reinform zu erhalten.

Geeignete Methoden sind z.B. Destillationsverfahren, wie fraktionierte Destillation unter Atmosphärendruck oder Anlegung eines Vakuums; oder Säulenchromatographie, wie eine SMB (simulated moving bed) Chromatographie oder CAC (continuous annular chromatography).

Die Aufreinigung kann z.B. batchwiese als auch kontinuierlich erfolgen.

Die Erfindung wird nun unter Bezugnahme auf folgende nichtlimitierende Ausführungsbeispiele näher erläutert:

### Experimenteller Teil

### Beispiel 1: Isolierung von (E/Z)-Hexadeca-8,15-dienal durch fraktionierte Destillation

2600 g eines Gemisches (Rohaustrag aus Oxidation von Cyclohexadeca-1,9-dien mit N₂O; wie z.B. beschrieben in der US20100191018) mit ca. 6% (E/Z)-Hexadeca-8,15-dienal wurden in einer Batchkolonne (Gewebepackung, 30 Stufen, Kopfdruck: 5 mbar, Druckverlust über Kolonne 5 mbar, Sumpftemperatur: 180°C, Sambayverdampfer, Rücklaufverhältnis: 100) fraktioniert destilliert. Das Gemisch aus (E/Z)-Hexadeca-8,15-dienal konnte dabei in verschiedenen Fraktionen auf 30, 37 und 65% angereichert werden.

### Beispiel 2: Synthese von Hexadeca-8,15-dienal durch N₂O-Oxidation von 1,9-Cyclohexadecadien

In einem adiabaten Rohrreaktor (3 m Länge, Durchmesser 6 cm, Reaktorvolumen 9 L) gefüllt mit Raschigringen aus 1.4541 Edelstahl wurden bei 216 °C Reaktoreingangstemperatur 2000 g/h 1,9-Cyclohexadecadien (*cis*/*trans*-Isomerengemisch) mit 52 mL/h einem N₂O/CO₂ Gemisch (15 % CO₂ Anteil) umgesetzt. Molverhältnis Olefin/N₂O/ 9-10. Nicht umgesetztes 1,9-Cyclohexadeca-1,9-dien wurde mittels einer Destillationskolonne (Montz-Gewebepackung A3, trennwirksame Höhe 4000 mm, Innendurchmesser 55 mm, Zulauf auf halber Höhe der Kolonne) bei 210 °C Sumpftemperatur und einem Kopfdruck von 20 mbar destillativ abgetrennt. Der Sumpfaustrag enthielt ca. 5 Gew.-% des Hexadeca-8,15-dienals (**I**) und weniger als 1 Gew.-% von 1,9-Cyclohexadecadien.

### Beispiel 3: Isolierung von (E/Z)-Hexadeca-8,15-dienal durch Säulenchromatographie

6 g eines Gemisches aus (E/Z)-Hexadeca-8,15-dienal (mind. 35%) (aus der fraktionierten Destillation von Beispiel 1) wurden mittels Säulenchromatographie unter Verwendung eines Laufmittelgemisches Cyclohexan/MTBE (50:1) gereinigt. Nach chromatographischer Reinigung wurden 1.8 g des Gemisches (E/Z)-Hexadeca-8,15-dienal mit einer Reinheit von 90% isoliert. Das isolierte Material wurde unter N₂-Atmosphäre gelagert.
¹H NMR (500 MHz, CDCl₃, 25 °C): σ = 9.8 (s, 1H), 5.85-5.75 (m, 1H), 5.4-5.3 (m, 2H), 4.9 (dd, 2H), 2.45-2.40 (m, 2H), 2.1-1.9 (m, 6H), 1.7-1.55 (m, 2H), 1.45-1.20 (m, 14H).
¹³C-NMR (125 MHz, CDCl₃, 25 °C): σ = 203 (2xCHO), 139.2, 139.1, 130.5, 130.1, 130.0, 129.6, 114.2 (C=CH₂), 114.1 (C=CH₂), 43.9 (2xCH₂), 33.8 (2xCH₂), 32.5 (2xCH₂), 29.6, 29.5 (2xCH₂), 29.3, 29.1, 29.0 (2xCH₂), 28.8 (3xCH₂), 28.8, 28.6, 27.1 (2xCH₂), 22.0 (2xCH₂).
IR (ATR) υ [cm⁻¹] = 3082, 3000, 2935, 2864, 2809, 2710, 1742, 1638, 1455, 994, 968, 724. MS m/z = 236, 165, 149, 121, 109, 95, 81, 67 (100), 55, 41.

### Beispiel 4: Olfaktorische Bewertung

Bewertet wurde eine (E)/(Z) Stoffgemisch isoliert durch Säulenchromatographie (Beispiel 2) Hexadeca-8,15-dienal ((E/Z)-Gemisch),

| | |
|---|---|
| Riechstreifentest <1min | grün, seifig, Moschuscharakter |
| Riechstreifentest 10min | seifig, schwach blumig, Moschuscharakter |
| Riechstreifentest 30min | seifig, schwach blumig, Moschuscharakter, aldehydisch |
| Riechstreifentest 1h | seifig, schwach blumig, Moschuscharakter, aldehydisch |
| Riechstreifentest 24h | moschusartig |

## Patentansprüche

1. Verwendung von Hexadeca-8,15-dienal der Formel I 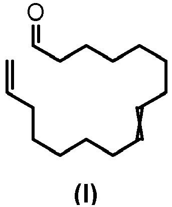 oder eines Stoffgemisches, welches diese Verbindung enthält als Aromachemikalie.

2. Verwendung nach Anspruch 1 in Mitteln, ausgewählt unter Parfüms, Wasch- und Reinigungsmitteln, kosmetischen Mitteln, Körperpflegemitteln, Hygieneartikeln, Lebensmitteln, Nahrungsergänzungsmitteln, Duftspendern, Duftstoffen, pharmazeutischen Mitteln und Pflanzenschutzmitteln.

3. Verwendung nach Anspruch 1 als Formulierungsmittel in Zubereitungen, die wenigstens eine Aromachemikalie enthalten.

4. Verwendung von Verbindungen der Formel (**I**) zum Vermitteln, Modifizieren und/oder Verstärken einer Moschusduftnote in einer Riechstoffzusammensetzung durch Beimischen einer sensorisch wirksamen Menge wenigstens eines Stoffs oder ein Stoffgemischs gemäß der Definition in Anspruch 1.

5. Verwendung nach einem der vorhergehenden Ansprüche, wobei die Verbindung der Formel (**I**) in stereoisomerenreiner Form oder als 8-(E)- und 8-(Z)-Gemisch eingesetzt wird.

6. Verwendung nach einem der vorhergehenden Ansprüche, wobei ein Stereoisomerengemisch eingesetzt wird, worin der Gewichtsanteil von 8-(E)- Hexadeca-8,15-dienal bezogen auf das Gesamtgewicht von (E)- und (Z)-Hexadeca-8,15-dienal in einem Bereich von 1% bis weniger als 100%, wie z.B. in einem Bereich von 25% bis 75% liegt.

7. Verfahren zur Isolierung von Hexadeca-8,15-dienal, wobei man aus einem Reaktionsgemisch aus der Oxidation von Cyclohexadeca-1,9-dien mit Lachgas, der Hexadeca-8,15-dienal enthält, ein (E/Z)-Gemisch von Hexadeca-8,15-dienal isoliert, wobei man Hexadeca-8,15-dienal als (E)- und (Z)-Gemisch mittels fraktionierter Destillation isoliert.

8. Verfahren nach Anspruch 7, wobei man Hexadeca-8,15-dienal als (E)- und (Z)-Gemisch aus einem Stoffgemisch isoliert, das eine Mischung aus (E/Z)-Hexadeca-8,15-dienal und Cyclohexadec-8-enon umfasst, worin der Gewichtsanteil von (E/Z)-Hexadeca-8,15-dienal, bezogen auf das Gesamtgewicht von (E/Z)-Hexadeca-8,15-dienal und Cyclohexadec-8-enon mindestens 5% beträgt.

9. Verfahren nach einem der Ansprüche 7 oder 8, wobei man Hexadeca-8,15-dienal als (E)- und (Z)-Gemisch mittels einer chromatographischen Methode isoliert, wobei man Hexadeca-8,15-dienal als (E)- und (Z)-Gemisch aus einem Stoffgemisch isoliert, worin das Gewichtsanteil von (E/Z)-Hexadeca-8,15-dienal, bezogen auf das Gesamtgewicht des Stoffgemisches mindestens 35% beträgt.

10. Mittel, enthaltend Hexadeca-8,15-dienal gemäß der Definition in einem der Ansprüche 1 bis 6, wobei das Mittel ausgewählt ist unter Parfüms, Wasch- und Reinigungsmitteln, kosmetischen Mitteln, Körperpflegemitteln, Hygieneartikeln, Lebensmitteln, Nahrungsergänzungsmitteln, Duftspendern, Duftstoffen, pharmazeutischen Mitteln und Pflanzenschutzmitteln.

11. Mittel nach Anspruch 10, enthaltend Hexadeca-8,15-dienal gemäß der Definition in einem der Ansprüche 1 bis 6 in einem Gewichtsanteil von 0,01 bis 99,9 Gew.-% bezogen auf das Gesamtgewicht der Zusammensetzung.

12. Verwendung von Hexadeca-8,15-dienal gemäß der Definition in einem der Ansprüche 1 bis 6 zum Vermitteln, Modifizieren und/oder Verstärken einer Moschusduftnote in einer Duft- oder Aromastoffzusammensetzung durch Beimischen einer sensorisch wirksamen Menge von des Hexadeca-8,15-dienals.

## Claims

1. The use of hexadeca-8,15-dienal of the formula I 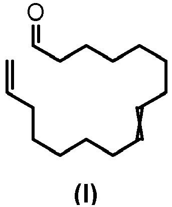 or of a substance mixture which comprises this compound as aroma chemical.

2. The use according to claim 1 in compositions selected from perfumes, detergents and cleaners, cosmetic compositions, bodycare compositions, hygiene articles, foods, food supplements, air fresheners, fragrances, pharmaceutical compositions and crop protection compositions.

3. The use according to claim 1 as formulation agent in preparations which comprise at least one aroma chemical.

4. The use of compounds of the formula (I) for conveying, modifying and/or intensifying a musk scent note in a fragrance composition by admixing a sensorally effective amount of at least one substance or of a substance mixture according to the definition in claim 1.

5. The use according to any one of the preceding claims, where the compound of the formula (I) is used in stereoisomerically pure form or as 8-(E) and 8-(Z) mixture.

6. The use according to any one of the preceding claims, where a stereoisomer mixture is used in which the weight fraction of 8-(E)-hexadeca-8,15-dienal, based on the total weight of (E)- and (Z)-hexadeca-8,15-dienal, is in a range from 1% to less than 100%, such as e.g. in a range from 25% to 75%.

7. A process for the isolation of hexadeca-8,15-dienal, where an (E/Z) mixture of hexadeca-8,15-dienal is isolated from a reaction mixture from the oxidation of cyclohexadeca-1,9-diene with nitrous oxide which comprises hexadeca-8,15-dienal, where hexadeca-8,15-dienal is isolated as (E) and (Z) mixture by means of fractional distillation.

8. The process according to claim 7, where hexadeca-8,15-dienal is isolated as (E) and (Z) mixture from a substance mixture which comprises a mixture of (E/Z)-hexadeca-8,15-dienal and cyclohexadec-8-enone, in which the weight fraction of (E/Z)-hexadeca-8,15-dienal, based on the total weight of (E/Z)-hexadeca-8,15-dienal and cyclohexadec-8-enone, is at least 5%.

9. The process according to either of claims 7 and 8, where hexadeca-8,15-dienal is isolated as (E) and (Z) mixture by means of a chromatographic method where hexadeca-8,15-dienal is isolated as (E) and (Z) mixture from a substance mixture in which the weight fraction of (E/Z)-hexadeca-8,15-dienal, based on the total weight of the substance mixture, is at least 35%.

10. A composition comprising hexadeca-8,15-dienal according to the definition in any one of claims 1 to 6 where the composition selected from perfumes, detergents and cleaners, cosmetic compositions, bodycare compositions, hygiene articles, foods, food supplements, air fresheners, fragrances, pharmaceutical compositions and crop protection compositions.

11. The composition according to claim 10, comprising hexadeca-8,15-dienal according to the definition in any one of claims 1 to 6 in a weight fraction of from 0.01 to 99.9% by weight, based on the total weight of the composition.

12. The use of hexadeca-8,15-dienal according to the definition in one of claims 1 to 6 for conveying, modifying and/or intensifying a musk scent note in a scent or aroma substance composition by admixing a sensorally effective amount of the hexadeca-8,15-dienal.

## Revendications

1. Utilisation d'hexadéca-8,15-diénal de formule I 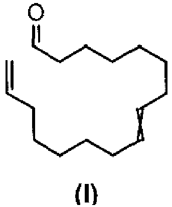 ou d'un mélange de substances, qui contient ce composé en tant qu'arôme chimique.

2. Utilisation selon la revendication 1 dans des agents, choisis parmi les parfums, les agents de lavage et de nettoyage, les agents cosmétiques, les agents de soin corporel, les articles hygiéniques, les aliments, les compléments alimentaires, les agents de distribution de parfum, les parfums, les agents pharmaceutiques et les agents de phytoprotection.

3. Utilisation selon la revendication 1 comme agent de formulation dans des préparations qui contiennent au moins un arôme chimique.

4. Utilisation de composés de formule (I) pour promouvoir, modifier et/ou renforcer une note parfumée de musc dans une composition odoriférante par mélange d'une quantité active d'un point de vue sensoriel d'au moins une substance ou d'un mélange de substances selon la définition dans la revendication 1.

5. Utilisation selon l'une quelconque des revendications précédentes, le composé de formule (I) étant utilisé sous une forme stéréo-isomère pure ou sous forme de mélange des formes 8-(E) et 8-(Z).

6. Utilisation selon l'une quelconque des revendications précédentes, un mélange de stéréo-isomères étant utilisé, dans lequel la proportion pondérale de 8-(E)-hexadéca-8,15-diénal par rapport au poids total de (E)-hexadéca-8,15-diénal et de (Z)-hexadéca-8,15-diénal se situe dans une plage de 1% à moins de 100%, comme par exemple dans une plage de 25% à 75%.

7. Procédé pour l'isolement de hexadéca-8,15-diénal, dans lequel on isole à partir d'un mélange réactionnel, provenant de l'oxydation de cyclohexadéca-1,9-diène avec du protoxyde d'azote, qui contient du hexadéca-8,15-diénal, un mélange (E/Z) d'hexadéca-8,15-diénal, l'hexadéca-8,15-diénal étant isolé en tant que mélange des formes (E) et (Z) par distillation fractionnée.

8. Procédé selon la revendication 7, dans lequel on isole de l'hexadéca-8,15-diénal sous forme de mélange des formes (E) et (Z) à partir d'un mélange de substances qui contient un mélange de (E/Z)-hexadéca-8,15-diénal et de cyclohexadéc-8-énone, la proportion pondérale de (E/Z)-hexadéca-8,15-diénal, par rapport au poids total de (E/Z)-hexadéca-8,15-diénal et de cyclohexadéc-8-énone étant d'au moins 5%.

9. Procédé selon l'une quelconque des revendications 7 ou 8, l'hexadéca-8,15-diénal étant isolé sous forme de mélange des formes (E) et (Z) au moyen d'un procédé chromatographique, l'hexadéca-8,15-diénal étant isolé sous forme de mélange des formes (E) et (Z) à partir d'un mélange de substances, dans lequel la proportion pondérale de (E/Z)-hexadéca-8,15-diénal, par rapport au poids total du mélange de substances, est d'au moins 35%.

10. Agent, contenant de l'hexadéca-8,15-diénal selon la définition dans l'une quelconque des revendications 1 à 6, l'agent étant choisi parmi les parfums, les agents de lavage et de nettoyage, les agents cosmétiques, les agents de soin corporel, les articles hygiéniques, les aliments, les compléments alimentaires, les agents de distribution de parfum, les parfums, les agents pharmaceutiques et les agents de phytoprotection.

11. Agent selon la revendication 10, contenant de l'hexadéca-8,15-diénal selon la définition dans l'une quelconque des revendications 1 à 6 en une proportion pondérale de 0,01 à 99,9% en poids par rapport au poids total de la composition.

12. Utilisation d'hexadéca-8,15-diénal selon la définition dans l'une quelconque des revendications 1 à 6 pour promouvoir, modifier et/ou renforcer une note parfumée de musc dans une composition odoriférante ou de substances aromatisantes par mélange d'une quantité active d'un point de vue sensoriel de l'hexadéca-8,15-diénal.
